# EUROPÄISCHE PATENTSCHRIFT

(11) **EP 2 528 661 B1**
(45) Veröffentlichungstag und Bekanntmachung des Hinweises auf die Patenterteilung: **11.01.2017**
(21) Anmeldenummer: 10773357.8
(22) Anmeldetag: 09.11.2010
(51) Int. Cl.: A61Q 5/10, A61K 8/44, A61K 8/04

(54) **SCHAUMFÖRMIGE FÄRBEMITTEL**
MOUSSE-TYPE DYES
COLORANTS SOUS FORME DE MOUSSE

(30) Priorität: 29.01.2010 DE 102010001375
(43) Veröffentlichungstag der Anmeldung: 05.12.2012
(73) Patentinhaber: Henkel AG & Co. KGaA, 40589 Düsseldorf (DE)
(72) Erfinder: ZIRWEN, Sabrina, 22089 Hamburg (DE); KLEEN, Astrid, 20457 Hamburg (DE); AKRAM, Mustafa, 22455 Hamburg (DE)
(86) Internationale Anmeldenummer: PCT/EP2010/067072
(87) Internationale Veröffentlichungsnummer: WO 2011/091875

(56) Entgegenhaltungen:
- EP-A1- 2 087 878
- WO-A1-98/36041
- JP-A- 2009 155 311
- US-A- 5 849 679
- US-A1- 2006 037 153
- US-A1- 2008 163 883
- US-B1- 6 283 336
- "International Cosmetic Ingredient Dictionary and Handbook", 2008, The Cosmetic, Toiletry, and Fragrance Association, XP002694060, Bd. 1, Seiten 639-640, Eintrag "COCO-BETAINE"

## Beschreibung

Die Erfindung betrifft Mittel zum Färben, die eine spezielles zwitterionisches Tensid und ein spezielles nichtionisches Tensid enthalten und geeignet sind, durch spezielle Applikationsvorrichtungen in Form eines stabilen Schaums aufgetragen zu werden, ein Färbeverfahren unter Zuhilfenahme der Mittel und der Applikationsvorrichtung sowie ein entsprechendes Kit zur Färbung von keratinhaltigen Fasern.

Für das Färben von keratinhaltigen Fasern kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet. Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt. Als Kupplerkomponenten werden in der Regel m-Phenylendiaminderivate, Naphthole, Resorcin und Resorcinderivate, Pyrazolone, m-Aminophenole und substituierte Pyridinderivate verwendet.

Zumeist besitzen oxidative Färbemittel zur Stabilisierung der Farbstoffvorprodukte während der Lagerung und zur Reaktionsbeschleunigung während der oxidativen Anwendung einen alkalischen pH-Wert, der mit Alkalisierungsmitteln, wie Alkanolaminen, Ammoniak oder anorganischen Basen, eingestellt wird. Insbesondere Ammoniak ermöglicht dabei zwar gute Färbeergebnisse, offenbart jedoch aufgrund seines Geruchs und Reizpotentials für Haut und Schleimhäute auch Nachteile für den Anwender. Daher liegen verstärkte Bemühungen auf der Entwicklung leistungsfähiger oxidativer Färbemittel, die auf den Einsatz von Ammoniak verzichten.

Oxidative Färbemittel bestehen üblicherweise aus zwei Komponenten, deren Mischung ausreichend zähflüssig ist, damit sie bequem auf die Haare aufgetragen werden kann und dabei nicht tropft oder verläuft. Darüber hinaus gab es häufig Versuche, andere Konfektionierungsformen zu entwickeln. So wurde vorgeschlagen, dünnflüssigere Färbemittel mit speziellen Applikatorsystemen auf die Haare aufzubringen oder Färbemittel als Schaum zu applizieren. Bei der Schaumapplikation ist insbesondere der Einsatz von Aerosolschäumen verbreitet.

Allerdings besteht in jüngerer Zeit auch das Bedürfnis, auf den Einsatz von Treibgasen verzichten zu können. Ein weiteres Problem bei der Schaumapplikation ist die Stabilisierung der Schäume. Die Beschaffenheit von Schäumen ist dann als ideal zu bezeichnen, wenn bei der Produktabgabe ein fester, stabiler Schaum entsteht, der ein geschmeidiges Gefühl hinterlässt und auf dem Haar nur langsam bricht. Häufig ist aber zu beobachten, dass die ausgebrachten Schäume wenig stabil sind und schnell wieder in sich zusammenfallen, so dass eine dünnflüssige, tropfende Lösung zurückbleibt. Andererseits ist es auch wesentlich, dass der Schaum trotzdem die Haare gut benetzt, so dass ein guter Farbauftrag stattfinden kann. Die Schaumstabilität wird insbesondere durch die Anwesenheit größerer Mengen an Salzen und Farbstoff(vorprodukt)en negativ beeinflusst. Aus der JP10-167938 A sind bereits schaumförmige Haarbehandlungsmittel bekannt, die als besonders geeignete Tenside für eine stabile Schaumbildung von sauren, wasserstoffperoxidhaltigen Haarbehandlungsmitteln betainische Tenside vom Typ der Alkyldimethylbetaine und der (Fettsäureamidoalkyl)dialkylbetaine wie Cocoa*midopropylbetain offenbaren. Cocoamidopropylbetain ist auch als geeignetes Tensid mit hohem Tensidgehalt in Tönungsschäumen auf Basis direktziehender Farbstoffe in WO 2006/066642(A1) beschrieben.

In EP 2 087 878 A1 werden oxidative Haarfärbe- und Blondierverfahren beschrieben, die auf zwei Komponenten basieren und aus einem Quetschbehälter in Form eines niedrig viskosen Schaums appliziert werden.

Es zeigte sich jedoch in umfangreichen Untersuchungen, dass gerade stark alkalische Oxidationsfarbstoffzubereitungen besondere Herausforderungen an das schaumbildende Tensid stellen. Insbesondere schäumende Oxidationsfarbstoffzubereitungen mit hohen Mengen an Fettsäureamidoalkylbetainen als schaumbildendes Tensid neigen, insbesondere in Gegenwart von Alkanolaminen als Alkalisierungsmittel, zur Entwicklung von Amin-artigen Gerüchen, die von Anwender als störend und unangenehm wahrgenommen werden.

Aufgabe der vorliegenden Erfindung war es daher, oxidative Färbemittel für die Schaumapplikation, ohne Verwendung von Treibgasen zu optimieren, so dass die oben genannten Nachteile überwunden werden können. Insbesondere sollten stabile Färbeschäume ohne unangenehme Gerüche, die möglichst auch ammoniakfrei sein sollen, bereit gestellt werden.

Es wurde überraschenderweise gefunden, dass oxidative Färbemittel, die ein spezielles, zwitterionisches Tensid sowie ein spezielles nichtionisches Tensid und ein Alkanolamin als Alkalisierungsmittel enthalten, zu äußerst stabilen Schäumen führen, die eine einfache und intensive Färbung der Fasern ermöglichen. Die Stabilität bleibt auch bei höheren Salzkonzentrationen erhalten. Außerdem ermöglichen die erfindungsgemäßen Mittel intensivere Farbergebnisse und deutlich haltbare Farbergebnisse als bisher bekannte Schaumzubereitungen. Schließlich sind die Mittel geruchsarm.

Ein erster Gegenstand der vorliegenden Erfindung sind daher Mittel zum Färben keratinischer Fasern, enthaltend in einem kosmetisch akzeptablen Träger mindestens ein Oxidationsfarbstoffvorprodukt sowie mindestens ein Alkanolamin, wobei das Mittel dadurch gekennzeichnet ist, dass das Mittel mindestens ein zwitterionisches Tensid der Formel (I), 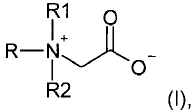 worin
- R: für eine gesättigte oder ungesättigte C₁₀-C₂₀-Alkylkette steht und
- R1 und R2: jeweils unabhängig voneinander für eine C₁-C₄-Alkylgruppe oder eine C₂-C₄-Hydroxyalkylgruppe stehen, und
mindestens ein nichtionisches Tensid enthält, wobei das Mittel als nichtionisches Tensid mindestens ein Alkylpolyglucosid und/oder Alkenylpolyglucosid enthält.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie beispielsweise Regeneratcellulose, Nitro-, Alkyl- oder Hydroxyalkyl- oder Acetylcellulose verwendet werden.

Die erfindungsgemäßen Mittel enthalten die Wirkstoffe in einem kosmetisch akzeptablen Träger. Dieser kosmetische Träger ist bevorzugt wässrig, alkoholisch oder wässrig-alkoholisch. Ein wässriger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten, sofern sie die Schaumbildung und -Stabilität nicht übermäßig negativ beeinflussen. Bevorzugt sind dabei alle wasserlöslichen organischen Lösungsmittel. Bevorzugte erfindungsgemäße Mittel sind dadurch gekennzeichnet, dass sie zusätzlich ein nichtwässriges Lösungsmittel enthalten, wobei bevorzugte erfindungsgemäße Mittel das Lösungsmittel in einer Konzentration von 0,1 bis 30 Gew.-%, bevorzugt in einer Konzentration von 1 bis 20 Gew.-%, ganz besonders bevorzugt in einer Konzentration von 2 bis 10 Gew.-%, jeweils bezogen auf das Mittel, enthalten.

Als erste erfindungswesentliche Komponente enthalten die Mittel mindestens ein Oxidationsfarbstoffvorprodukt. Vorzugsweise enthält das Mittel eine oder mehrere Entwicklerkomponenten sowie gegebenenfalls eine oder mehrere Kupplerkomponenten.

Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 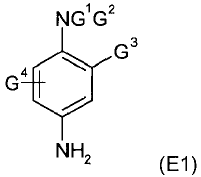 wobei
- G¹ steht für ein Wasserstoffatom, einen C₁-C₄-Alkylrest, einen C₁-C₄)-Monohydroxyalkylrest, einen C₂-C₄-Polyhydroxyalkylrest, einen C₁-C₄-Alkoxy-(C₁-C₄)-alkylrest, einen 4-Aminophenylrest oder einen C₁-C₄-Alkylrest, der mit einer stickstoffhaltigen Gruppe, einem Phenyl- oder einem 4-Aminophenylrest substituiert ist;
- G² steht für ein Wasserstoffatom, einen C₁-C₄-Alkylrest, einen C₁-C₄-Monohydroxyalkylrest, einen C₂-C₄-Polyhydroxyalkylrest, einen C₁-C₄-Alkoxy-(C₁-C₄)-alkylrest oder einen C₁-C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁-C₄-Alkylrest, einen C₁-C₄-Monohydroxyalkylrest, einen C₂-C₄-Polyhydroxyalkylrest, einen C₁-C₄-Hydroxyalkoxyrest, einen C₁-C₄-Alkoxy-(C₁-C₄)-alkylrest, einen C₁-C₄-Acetylaminoalkoxyrest, einen Mesylamino-(C₁-C₄)-alkoxyrest oder einen C₁-C₄-Carbamoylaminoalkoxyrest;
- G⁴ steht für ein Wasserstoffatom, ein Halogenatom, einen C₁-C₄-Alkylrest oder einen C₁-C₄-Alkoxy-(C₁-C₄)-alkylrest oder
- wenn G³ und G⁴ in ortho-Stellung zueinander stehen, können sie gemeinsam eine verbrückende α,ω)-Alkylendioxogruppe, wie beispielsweise eine Ethylendioxygruppe bilden.

Besonders bevorzugte p-Phenylendiamine der Formel (E1) werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(2-hydroxyethyl)-p-phenylendiamin, 4-N,N-Bis-(2-hydroxyethyl)-amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N,N-(Ethyl,2-hydroxyethyl)-p-phenylendiamin, N-(2,3-Dihydroxypropyl)-p-phenylendiamin, N-(4-Aminophenyl)-p-phenylendiamin, N-Phenyl-p-phenylendiamin, 2-(2-Hydroxy-ethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß ganz besonders bevorzugte p-Phenylendiaminderivate der Formel (E1) sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie deren physiologisch verträglichen Salze.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze, 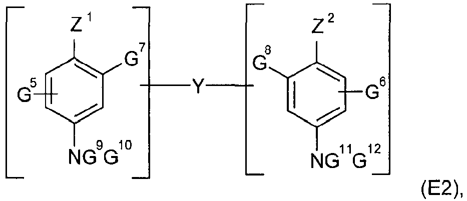 wobei
- Z¹ und Z² stehen unabhängig voneinander für einen Hydroxyl- oder NH₂-Rest, der gegebenenfalls durch einen C₁-C₄-Alkylrest, durch einen C₁-C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁-C₈-Alkoxyreste substituiert sein kann, oder eine direkte Bindung,
- G⁵ und G⁶ stehen unabhängig voneinander für ein Wasserstoff- oder Halogenatom, einen C₁-C₄-Alkylrest, einen C₁-C₄-Monohydroxyalkylrest, einen C₂-C₄-Polyhydroxyalkylrest, einen C₁-C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁-C₄-Alkylrest,
mit der Maßgabe, dass die Verbindungen der Formel (E2) nur eine Verbrückung Y pro Molekül enthalten.

Bevorzugte zweikernige Entwicklerkomponenten der Formel (E2) werden insbesondere aus mindestens einer der folgenden Verbindungen ausgewählt: N,N'-Bis-(2-hydroxyethyl)-N,N'-bis-(4-aminophenyl)-1,3-diamino-propan-2-ol, N,N'-Bis-(2-hydroxyethyl)-N,N'-bis-(4-aminophenyl)ethylendiamin, N,N'-Bis-(4-aminophenyl)tetramethylendiamin, N,N'-Bis-(2-hydroxyethyl)-N,N'-bis-(4-aminophenyl)tetramethylendiamin, N,N'-Bis-(4-(methylamino)phenyl)tetramethylendiamin, N,N'-Diethyl-N,N'-bis-(4-amino-3-methylphenyl)ethylendiamin, Bis-(2-hydroxy-5-aminophenyl)methan, N,N'-Bis-(4-aminophenyl)-1,4-diazacycloheptan, N,N'-Bis-(2-hydroxy-5-aminobenzyl)piperazin, N-(4-Aminophenyl)-p-phenylendiamin und 1,10-Bis-(2',5'-diaminophenyl)-1,4,7,10-tetraoxadecan sowie ihre physiologisch verträglichen Salze. Ganz besonders bevorzugte zweikernige Entwicklerkomponenten der Formel (E2) werden ausgewählt unter N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diamino-phenyl)-1,4,7,10-tetraoxadecan oder eines deren physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3), 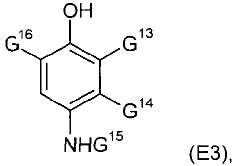 wobei
- G¹³ steht für ein Wasserstoffatom, ein Halogenatom, einen C₁-C₄-Alkylrest, einen C₁-C₄-Monohydroxyalkylrest, einen C₂-C₄-Polyhydroxyalkylrest, einen C₁-C₄-Alkoxy-(C₁-C₄)-alkylrest, einen C₁-C₄-Aminoalkylrest, einen Hydroxy-(C₁-C₄)-alkylaminorest, einen C₁-C₄-Hydroxyalkoxyrest, einen C₁-C₄-Hydroxyalkyl-(C₁-C₄)-aminoalkylrest oder einen Di-[(C₁-C₄)-alkyl]amino-(C₁-C₄)-alkylrest, und
- G¹⁴ steht für ein Wasserstoff- oder Halogenatom, einen C₁-C₄-Alkylrest, einen C₁-C₄-Mono-hydroxyalkylrest, einen C₂-C₄-Polyhydroxyalkylrest, einen C₁-C₄-Alkoxy-(C₁-C₄)-alkylrest, einen C₁-C₄-Aminoalkylrest oder einen C₁-C₄-Cyanoalkylrest,
- G¹⁵ steht für Wasserstoff, einen C₁-C₄-Alkylrest, einen C₁-C₄-Monohydroxyalkylrest, einen C₂-C₄-Polyhydroxyalkylrest, einen Phenylrest oder einen Benzylrest, und
- G¹⁶ steht für Wasserstoff oder ein Halogenatom.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-amino-methylphenol, 4-Amino-2-(2-hydroxyethylaminomethyl)phenol, 4-Amino-2-(1,2-dihydroxyethyl)-phenol, 4-Amino-2-fluorphenol, 4-Amino-2-chlorphenol, 4-Amino-2,6-dichlorphenol, 4-Amino-2-(di-ethylaminomethyl)phenol sowie ihre physiologisch verträglichen Salze. Besonders bevorzugte Verbindungen der Formel (E3) sind p-Aminophenol, 4-Amino-3-methylphenol, 4-Amino-2-amino-methylphenol, 4-Amino-2-(1,2-dihydroxyethyl)phenol und 4-Amino-2-(diethylaminomethyl)phenol. Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol. Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen. Besonders bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Besonders bevorzugte Pyrazol-Derivate sind insbesondere die Verbindungen, die ausgewählt werden unter 4,5-Diamino-1-methylpyrazol, 4,5-Diamino-1-(β-hydroxyethyl)-pyrazol, 3,4-Diaminopyrazol, 4,5-Diamino-1-(4-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol sowie deren physiologisch verträglichen Salze. Bevorzugte Pyrazolopyrimidine sind Pyrazolo[1,5-a]-pyrimidine. Unter den Pyrazolo[1,5-a]pyrimidinen sind wiederum Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethyl-pyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)-(2-hydroxyethyl)-amino]-ethanol, 2-[(7-Aminopyrazolo[1,5-a]pyrimidin-3-yl)-(2-hydroxy-ethyl)-amino]-ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihre physiologisch verträglichen Salze und ihre tautomeren Formen, wenn ein tautomers Gleichgewicht vorhanden ist, besonders bevorzugt.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin sowie deren physiologisch verträglichen Salzen.

Die Entwicklerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Diese Gruppen stehen durch ein Doppelbindungssystem in Konjugation.

Erfindungsgemäße Kupplerkomponenten werden bevorzugt ausgewählt den Klassen von m-Aminophenol und/oder dessen Derivaten, m-Diaminobenzol und/oder dessen Derivaten, o-Diaminobenzol und/oder dessen Derivaten, Naphthalinderivaten mit mindestens einer Hydroxygruppe, Di- beziehungsweise Trihydroxybenzol und/oder deren Derivaten, Pyridinderivaten, Pyrimidinderivaten, Monohydroxyindol-Derivaten und/oder Monoaminoindol-Derivaten, Monohydroxyindolin-Derivaten und/oder Monoaminoindolin-Derivaten, Pyrazolonderivaten, wie 1-Phenyl-3-methylpyrazol-5-on, Morpholinderivate, wie 6-Hydroxybenzomorpholin oder 6-Aminobenzomorpholin, Chinoxalinderivaten, wie 6-Methyl-1,2,3,4-tetrahydrochinoxalin, sowie Gemischen aus zwei oder mehrer Verbindungen aus einer oder mehrerer dieser Klassen.

Bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol, 2,4-Dichlor-3-aminophenol und deren physiologisch verträglichen Salzen. Bevorzugte m-Diaminobenzol-Kupplerkomponenten werden ausgewählt aus mindestens einer Verbindung aus der Gruppe, die gebildet wird aus 2-(2,4-Diaminophenoxy)ethanol, 1,3-Bis-(2,4-diaminophenoxy)propan, 1-Methoxy-2-amino-4-(2'-hydroxyethylamino)benzol, 1,3-Bis-(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methyl-phenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol und deren physiologisch verträglichen Salzen. Bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und deren physiologisch verträglichen Salzen. Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol. Bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 2,6-Dihydroxy-3,4-dimethylpyridin, 3,4-Diaminopyridin, 2-(2-Methoxyethyl)amino-3-amino-6-methoxypyridin, 2-(4'-Methoxyphenyl)amino-3-aminopyridin und deren physiologisch verträglichen Salzen. Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin. Bevorzugte Indolderivate werden ausgewählt aus mindestens einer Verbindung der Gruppe, die gebildet wird aus 4-Hydroxyindol, 6-Hydroxyindol und 7-Hydroxyindol und deren physiologisch verträglichen Salzen. Bevorzugte Indolinderivate werden ausgewählt aus mindestens einer Verbindung der Gruppe, die gebildet wird aus 4-Hydroxyindolin, 6-Hydroxyindolin und 7-Hydroxyindolin und deren physiologisch verträglichen Salzen. Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen.

Erfindungsgemäß besonders bevorzugte Kupplerkomponenten werden ausgewählt unter m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2-Hydroxy-ethyl)amino-2-methylphenol, 3-(Diethylamino)phenol, N-Cyclopentyl-3-aminophenol, 1,3-Dihydroxy-5-(methylamino)benzol, 3-Ethylamino-4-methylphenol, 2,4-Dichlor-3-aminophenol, 2-(2,4-Diaminophenoxy)ethanol, 1,3-Bis-(2,4-diaminophenoxy)propan, 1-Methoxy-2-amino-4-(2-hydroxy-ethylamino)benzol, 1,3-Bis-(2,4-diaminophenyl)propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxy-ethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, Resorcinmonomethylether, 2-Methylresorcin, 5-Methylresorcin, 2,5-Dimethylresorcin, 2-Chlorresorcin, 4-Chlorresorcin, Pyrogallol, 1,2,4-Trihydroxybenzol, 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 2,6-Dihydroxy-3,4-dimethylpyridin, 3,4-Diaminopyridin, 2-(2-Methoxyethyl)amino-3-amino-6-methoxypyridin, 2-(4'-Methoxyphenyl)amino-3-aminopyridin, 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin, 2,3-Dihydroxynaphthalin, 4-Hydroxyindol, 6-Hydroxyindol, 7-Hydroxyindol, 4-Hydroxyindolin, 6-Hydroxyindolin, 7-Hydroxyindolin, 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin oder Gemischen dieser Verbindungen oder deren physiologisch verträglichen Salzen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Zur weiteren Nuancierung der resultierenden Farbtöne kann es erfindungsgemäß bevorzugt sein, dem Mittel weiterhin mindestens einen direktziehenden Farbstoff zuzusetzen. Hierbei handelt es sich um Farbstoffmoleküle, die direkt auf das Substrat aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Heutzutage sind direktziehende Farbstoffe üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.%. Direktziehende Farbstoffe können in anionische, kationische und nichtionische direktziehende Farbstoffe unterteilt werden.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 sowie Tetrabromphenolblau und Bromphenolblau bekannten Verbindungen.

Bevorzugte kationische direktziehende Farbstoffe sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden. Die Verbindungen, die auch unter den Bezeichnungen Basic Yellow 87, Basic Orange 31 und Basic Red 51 bekannt sind, sind ganz besonders bevorzugte kationische direktziehende Farbstoffe.

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu und Alkannawurzel enthalten sind.

Weiterhin hat es sich als vorteilhaft erwiesen, wenn die erfindungsgemäßen Mittel zur weiteren Nuancierung zusätzlich eine oder mehrere Farbstoffvorstufen naturanaloger Farbstoffe enthalten. Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Besonders gut als Vorstufen naturanaloger Haarfarbstoffe geeignet sind Derivate des 5,6-Dihydroxyindolins. Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure. Als Vorstufen naturanaloger Haarfarbstoffe hervorragend geeignet sind weiterhin Derivate des 5,6-Dihydroxyindols. Bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure.

Als zweite erfindungswesentliche Komponente enthalten die erfindungsgemäßen Mittel als Alkalisierungsmittel mindestens ein Alkanolamin.

Erfindungsgemäß einsetzbare Alkanolamine werden bevorzugt ausgewählt aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird aus 2-Aminoethan-1-ol (Monoethanolamin), 3-Aminopropan-1-ol, 4-Amino-butan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Amino-propan-1,2-diol, 2-Amino-2-methylpropan-1,3-diol, 2-Amino-2-ethyl-1,3-propandiol, N,N-Dimethylethanolamin, Methylglucamin, Triethanolamin, Diethanolamin und Triisopropanolamin. Erfindungsgemäß ganz besonders bevorzugte Alkanolamine werden ausgewählt aus der Gruppe 2-Amino-ethan-1-ol (Monoethanolamin), 2-Amino-2-methylpropan-1-ol, 2-Amino-2-methyl-propan-1,3-diol und Triethanolamin. Besonders bevorzugte Mittel enthalten als Alkanolamin mindestens Monoethanolamin.

Bevorzugt sind die Alkanolamine in einer Menge von 0,05 bis 20 Gew.-%, insbesondere von 0,5 bis 15 Gew.-%, jeweils bezogen auf das Gesamtgewicht des anwendungsbereiten Mittels, enthalten.

Als dritte erfindungswesentliche Komponente enthalten die erfindungsgemäßen Färbemittel mindestens ein zwitterionisches Tensid der Formel (I), 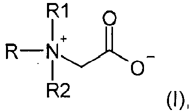 worin
- R: für eine gesättigte oder ungesättigte C₁₀-C₂₀-Alkylkette steht und
- R1 und R2: jeweils unabhängig voneinander für eine C₁-C₄-Alkylgruppe oder eine C₂-C₄-Hydroxyalkylgruppe stehen.

Der Rest R steht dabei für eine Alkylkette mit 10 bis 20, vorzugsweise 10 bis 18 Kohlenstoffatomen, die eine oder mehrere Doppelbindungen ausweisen kann und gegebenenfalls verzweigt sein kann. Bevorzugte Beispiele solcher Alkylgruppen sind die Decyl-, Lauryl-, Myristyl-, Cetyl-, Palmoleyl-, 2-Hexyldecyl-, Stearyl-, Isostearyl-, Oleyl-, Elaidyl-, Petroselinyl-, Arachyl-, 2-Octyldodecyl oder Gadoleyl-Gruppe sowie deren Gemische, wie sie aufgrund von eingesetztem Rohstoff oder Herstellmethode anfallen. Bevorzugt sind für R Alkylgruppen auf Basis von Cocosalkyl- oder Talgfettalkylgruppen.

Die Reste R1 und R2 stehen unabhängig voneinander für eine C₁-C₄-Alkylgruppe oder eine C₂-C₄- Hydroxyalkylgruppe. Geeignete und bevorzugte C₁-C₄-Alkylgruppen sind dabei die Methyl-, Ethyl-, Propyl,- Isopropyl-, Butyl-, 1-Methylpropyl-, 2-Methylpropyl- und 1,1-Dimethylethyl-Gruppe. Geeignete und bevorzugte C₂-C₄-Hydroxyalkylgruppen sind dabei die 2-Hydroxyethyl-, 3-Hydroxy-propyl- und 2-Hydroxypropyl-Gruppe. Besonders bevorzugt stehen R1 und R2 jeweils für eine Methylgruppe.

Eine Ausführungsform des ersten Erfindungsgegenstands ist dadurch gekennzeichnet, dass das Mittel als zwitterionisches Tensid mindestens eine Verbindung der Formel (I) enthält, worin R1 und R2 jeweils für eine Methylgruppe und R für eine Cocosalkylgruppe stehen.

Solche besonders geeigneten zwitterionischen Tenside tragen die INCI-Bezeichnung Coco Betaine und werden beispielsweise als wässrige Lösung mit 30 Gew.-% Aktivsubstanz unter dem Handelsnamen Genagen^{®} KB vertrieben.

Um einen stabilen Schaum zu erzeugen, ist es notwendig, dass das anwendungsbereite Mittel eine ausreichende Menge an zwitterionischem Tensid enthält. Eine Ausführungsform des ersten Erfindungsgegenstands ist daher dadurch gekennzeichnet, dass das Mittel ein oder mehrere zwitterionische Tenside der Formel (I) in einem Gesamtgewichtsanteil von mindestens 2,5 Gew.-%, bevorzugt von mindestens 3 Gew.-% und insbesondere bevorzugt von mindestens 4 Gew.-%, bezogen auf das Gesamtgewicht des anwendungsbereiten Mittels, enthält.

Um die Schaumbildung und Schaumstabilität weiter zu verbessern, wird dem Mittel als vierte erfindungswesentliche Komponente mindestens ein nichtionisches Tensid zugesetzt, wobei dem Mittel als nichtionisches Tensid mindestens ein Alkylpolyglucosid und/oder Alkenylpolyglucosid zugesetzt werden.

Als erfindungsgemäße nichtionische Tenside haben sich Alkylpolyglykoside sowie Alkylenoxid-Anlagerungsprodukte an gesättigte lineare Fettalkohole, Fettsäureester und Fettsäuren mit jeweils 2 bis 80 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside zusätzlich Fettsäureester von ethoxyliertem Glycerin enthalten.
Gemäß einer erfindungsgemäßen Ausführungsform enthält das Mittel als nichtionisches Tensid mindestens ein Alkyl- und/oder Alkenylpolyglucosid. Alk(en)ylpolyglucoside stellen bekannte nichtionische Tenside gemäß Formel (II) dar,

R¹O-[G]p (II)

in der R¹ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht.

Die Alkyl- und Alkenylpolyglucoside können sich von Aldosen beziehungsweise Ketosen mit 5 oder 6 Kohlenstoffatomen, vorzugsweise von Glucose, ableiten. Die bevorzugten Alkyl- und/oder Alkenylpolyglucoside sind somit Alkyl- und/oder Alkenylpolyglucoside. Die Indexzahl p in der allgemeinen Formel (II) gibt den Polymerisierungsgrad (DP), d. h. die Verteilung von Mono- und Polyglucosiden an und steht für eine Zahl zwischen 1 und 10. Während p im einzelnen Molekül stets ganzzahlig sein muss und hier vor allem die Werte p = 1 bis 6 annehmen kann, ist der Wert p für ein bestimmtes Alkylpolyglucosid eine analytisch ermittelte rechnerische Größe, die meistens eine gebrochene Zahl ist. Vorzugsweise werden Alkyl- und/oder Alkenylpolyglucoside mit einem mittleren Oligomerisierungsgrad p von 1,1 bis 3,0 eingesetzt. Aus anwendungstechnischer Sicht sind solche Alkyl- und/oder Alkenylpolyglucoside bevorzugt, deren Polymerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkylpolyglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkylpolyglucoside auf Basis technischer C_{9/11}-Oxoaikohoie (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkylpolyglucoside auf Basis von gehärtetem C_{12/14}-Kokosalkohol mit einem DP von 1 bis 3.

Erfindungsgemäß geeignete Alkyl- und/oder Alkenylpolyglucoside werden unter der INCl-Bezeichnung Coco-Glucoside und dem Handelsnamen Plantacare 818 UP oder unter der INCl-Bezeichnung Lauryl-Glucoside und dem Handelsnamen Plantacare 1200 UP vertrieben.

Das beziehungsweise die Alkyl- und/oder Alkenylpolyglucoside sind in den erfindungsgemäßen Mitteln bevorzugt in Mengen von 0,1 bis 20 Gew.-%, bezogen auf das anwendungsbereite Mittel, enthalten. Mengen von 1 bis 15 Gew.-% sind besonders bevorzugt. Mengen von 3 bis 8 Gew.-% sind ganz besonders bevorzugt.

Weiterhin hat es sich als vorteilhaft erwiesen, wenn die erfindungsgemäßen Mittel mindestens ein weiteres nichtionisches Tensid enthalten, das von den Alk(en)ylpolyglucosiden verschieden ist. Bevorzugt werden dabei ethoxylierte, nichtionische Tenside eingesetzt

Dabei hat es sich als besonders vorteilhaft erwiesen, wenn das weitere nichtionische Tensid einen HLB-Wert oberhalb von 10, vorzugsweise oberhalb von 14 aufweist. Dazu ist notwendig, dass das nichtionische Tensid einen ausreichend hohen Ethoxylierungsgrad aufweist.

Eine weitere Ausführungsform des ersten Erfindungsgegenstands ist daher dadurch gekennzeichnet, dass das Mittel als nichtionisches Tensid mindestens ein ethoxyliertes Tensid mit mindestens 30 Ethylenoxideinheiten enthält.

Neben den entsprechend ethoxylierten Fettalkoholen sind erfindungsgemäß insbesondere die Anlagerungsprodukte von 30 bis 60 Mol Ethylenoxid an Rizinusöl und gehärtetes Rizinusöl besonders geeignet. Diese speziellen nichtionischen Tenside ermöglichen eine weitere Verbesserung der Schaumkonsistenz, insbesondere im Hinblick auf eine höhere Festigkeit, eine gesteigerte Feinporigkeit und eine größere Geschmeidigkeit.

Beispiele für solche geeigneten Tenside tragen die INCl-Bezeichnungen Steareth-30. Ceteareth-30, Oleth-30, Ceteareth-50 oder PEG-40 Hydrogenated Castor Oil und PEG-60 Hydrogenated Castor Oil. PEG-60 Hydrogenated Castor Oil wird beispielsweise unter dem Handelsnamen Cremophor CO 60 vertrieben.

Das beziehungsweise die ethoxylierten, nichtionischen Tenside sind in den erfindungsgemäßen Mitteln bevorzugt in Mengen von 0,1 bis 10 Gew.-%, bevorzugt von 0,5 bis 8 Gew.-% und besonders bevorzugt von 1,0 bis 5,0 Gew.-% , jeweils bezogen auf das anwendungsbereite Mittel, enthalten.

Weiterhin hat es sich als erfindungsgemäß als wesentlich zur Erzielung der gewünschten Schaumeigenschaften erwiesen, wenn das anwendungsbereite Mittel einen Gesamttensidgehalt von mindestens 10 Gew.-% aufweist. Mittel, die mindestens 11 Gew.-%, vorzugsweise mindestens 12 Gew.-% Gesamttensidgehalt, jeweils bezogen auf das anwendungsbereite Mittel, aufweisen sind insbesondere bevorzugt.

Außerdem hat es sich als vorteilhaft erwiesen, wenn die Anwendungszubereitung frei von kationischen Tensiden formuliert ist.

Ferner konnte in den dieser Erfindung zugrunde liegenden Arbeiten gezeigt werden, dass es vorteilhaft ist, wenn die Mittel neben den oben genannten Tensiden mindestens ein anionisches Tensid aufweisen. Als anionische Tenside sind
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist, und
- Alkylsulfate und Alkylpolyglycolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
erfindungsgemäß besonders bevorzugt.

In einer ganz besonders bevorzugten Ausführungsform der vorliegenden Erfindung enthalten die Mittel neben den erfindungswesentlichen Tensiden weiterhin ein anionisches Tensid. Insbesondere bei zweiteiligen Mitteln, bei denen farbstoffhaltigen Komponente und Oxidationsmittel getrennt konfektioniert sind, es sich als vorteilhaft erwiesen, wenn das anionische Tensid in der Oxidationsmittelzubereitung und die übrigen Tenside in der farbstoffhaltigen Komponente enthalten sind.

Überraschenderweise konnte gezeigt werden, dass die Anwesenheit geringer Mengen eines polymeren Verdickers die Schaumbeständigkeit positiv beeinflussen konnte. Daher ist es im Rahmen einer Ausführungsform der vorliegenden Erfindung bevorzugt, wenn die Mittel mindestens einen polymeren Verdicker enthalten.

Beispiele für erfindungsgemäß bevorzugte polymere Verdicker sind: Acrylates Copolymer, Acrylamides Copolymer, Acrylamide/Sodium Acrylate Copolymer, Acrylamide/Sodium Acryloyldimethyltaurate Copolymer, Acrylates/Acetoacetoxyethyl Methacrylate Copolymer, Acrylates/Beheneth-25 Methacrylate Copolymer, Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, Acryiates/Vinyl Isodecanoate Crosspolymer, Acrylic Acid/Acrylonitrogens Copolymer, Agar, Agarose, Alcaligenes Polysaccharides, Algin, Alginic Acid, Ammonium Acrylates/Acrylonitrogens Copolymer, Ammonium Acrylates Copolymer, Ammonium Acryloyldimethyltaurate/Vinyl Formamide Copolymer, Ammonium Acryioyidimethyitaurate/VP Copolymer, Ammonium Alginate, Ammonium Polyacryloyldimethyl Taurate, Amylopectin, Ascorbyl Methylsilanol Pectinate, Astragalus Gummifer Gum, Attapulgite, Avena Sativa (Oat) Kernel Flour, Bentonite, Butoxy Chitosan, Caesalpinia Spinosa Gum, Calcium Alginate, Calcium Carboxymethyl Cellulose, Calcium Carrageenan, Calcium Potassium Carbomer, Calcium Starch Octenylsuccinate, C20-40 Alkyl Stearate, Carbomer, Carboxybutyl Chitosan, Carboxymethyl Chitin, Carboxymethyl Chitosan, Carboxymethyl Dextran, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Cholesterol/HDI/Pullulan Copolymer, Cholesteryl Hexyl Dicarbamate Pullulan, Cyamopsis Tetragonoloba (Guar) Gum, Diglycol/CHDM/Isophthalates/SIP Copolymer, Dihydrogenated Tallow Benzylmonium Hectorite, Dimethicone Crosspolymer-2, Dimethicone Propyl PG-Betaine, DMAPA Acrylates/Acrylic Acid/Acrylonitrogens Copolymer, Ethylene/Sodium Acrylate Copolymer, Gelatin, Gellan Gum, Glyceryl Alginate, Glycine Soja (Soybean) Flour, Guar Hydroxypropyltrimonium Chloride, Hectorite, Hydrated Silica, Hydrogenated Potato Starch, Hydroxybutyl Methylcellulose, Hydroxyethyl Acrylate/Sodium Acryloyldimethyl Taurate Copolymer, Hydroxyethylcellulose, Hydroxyethyl Chitosan, Hydroxyethyl Ethylcellulose, Hydroxypropylcellulose, Hydroxypropyl Chitosan, Hydroxypropyl Ethylenediamine Carbomer, Hydroxypropyl Guar, Hydroxypropyl Methylcellulose, Hydroxypropyl Methylcellulose Stearoxy Ether, Hydroxypropyl Starch, Hydroxypropyl Starch Phosphate, Hydroxypropyl Xanthan Gum, Hydroxystearamide MEA, Isobutylene /Sodium Maleate Copolymer, Lithium Magnesium Silicate, Lithium Magnesium Sodium Silicate, Macrocystis Pyrifera (Kelp), Magnesium Alginate, Magnesium Aluminum Silicate, Magnesium Silicate, Magnesium Trisilicate, Methoxy PEG-22/Dodecyl Glycol Copolymer, Methylcellulose, Methyl Ethylcellulose, Methyl Hydroxyethylcellulose, Microcrystalline Cellulose, Montmorillonite, Moroccan Lava Clay, Natto Gum, Nonoxynyl Hydroxyethylcellulose, Octadecene/MA Copolymer, Pectin, PEG-800, PEG-Crosspolymer, PEG-150/Decyl Alcohol/SMDI Copolymer, PEG-175 Diisostearate, PEG-190 Distearate, PEG-15 Glyceryl Tristearate, PEG-140 Glyceryl Tristearate, PEG-240/HDI Copolymer Bis-Decyltetradeceth-20 Ether, PEG-100/IPDI Copolymer, PEG-180/Laureth-50/TMMG Copolymer, PEG-10/Lauryl Dimethicone Crosspolymer, PEG-15/Lauryl Dimethicone Crosspolymer, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG-120 Methyl Glucose Trioleate, PEG-180/Octoxynol-40/TMMG Copolymer, PEG-150 Pentaerythrityl Tetrastearate, PEG-4 Rapeseedamide, PEG-150/Stearyl Alcohol/SMDI Copolymer, Polyacrylate-3, Polyacrylic Acid, Polycyclopentadiene, Polyether-1, Polyethylene/Isopropyl Maleate/MA Copolyol, Polymethacrylic Acid, Polyquaternium-52, Polyvinyl Alcohol, Potassium Alginate, Potassium Aluminum Polyacrylate, Potassium Carbomer, Potassium Carrageenan, Potassium Polyacrylate, Potato Starch Modified, PPG-14 Laureth-60 Hexyl Dicarbamate, PPG-14 Laureth-60 Isophoryl Dicarbamate, PPG-14 Palmeth-60 Hexyl Dicarbamate, Propylene Glycol Alginate, PVP/Decene Copolymer, PVP Montmorillonite, Rhizobian Gum, Ricinoleic Acid/Adipic Acid/AEEA Copolymer, Sclerotium Gum, Sodium Acrylate/Acryloyldimethyl Taurate Copolymer, Sodium Acrylates/Acrolein Copolymer, Sodium Acrylates/Acrylonitrogens Copolymer, Sodium Acrylates Copolymer, Sodium Acrylates /Vinyl Isodecanoate Crosspolymer, Sodium Acrylate/Vinyl Alcohol Copolymer, Sodium Carbomer, Sodium Carboxymethyl Chitin, Sodium Carboxymethyl Dextran, Sodium Carboxymethyl Beta-Glucan, Sodium Carboxymethyl Starch, Sodium Carrageenan, Sodium Cellulose Sulfate, Sodium Cyclodextrin Sulfate, Sodium Hydroxypropyl Starch Phosphate, Sodium Isooctylene/MA Copolymer, Sodium Magnesium Fluorosilicate, Sodium Polyacrylate, Sodium Polyacrylate Starch, Sodium Polyacryloyldimethyl Taurate, Sodium Polymethacrylate, Sodium Polystyrene Sulfonate, Sodium Silicoaluminate, Sodium Starch Octenylsuccinate, Sodium Stearoxy PG-Hydroxyethylcellulose Sulfonate, Sodium Styrene/Acrylates Copolymer, Sodium Tauride Acrylates/Acrylic Acid/ Acrylonitrogens Copolymer, Solanum Tuberosum (Potato) Starch, Starch/Acrylates/Acrylamide Copolymer, Starch Hydroxypropyltrimonium Chloride, Steareth-60 Cetyl Ether, Steareth-100/PEG-136 / HDI Copolymer, Sterculia Urens Gum, Synthetic Fluorphlogopite, Tamarindus Indica Seed Gum, Tapioca Starch, TEA-Alginate, TEA-Carbomer, Triticum Vulgare (Wheat) Starch, Tromethamine Acrylates/Acrylonitrogens Copolymer, Tromethamine Magnesium Aluminum Silicate, Welan Gum, Xanthan Gum, Yeast Beta-Glucan, Yeast Polysaccharides, Zea Mays (Corn) Starch.

Aus dieser umfangreichen Gruppe haben sich die Verdickungsmittel als besonders vorteilhaft erwiesen, die mindestens ein Monomer vom Typ der Acrylsäure oder Methacrylsäure sowie derer Derivate enthalten. Ein erfindungsgemäß ganz besonders bevorzugtes Polymer ist das unter der INCI-Bezeichnung Acrylates Copolymer bekannte Copolymer aus zwei oder mehr Monomeren, ausgewählt aus Acrylsäure, Methacrylsäure und deren Ester mit C₁-C₄-Alkylgruppen.

Wie bereits ausgeführt, ist es besonders vorteilhaft, wenn der polymere Verdicker in geringen Mengen, vorzugsweise in Mengen von 0,05 bis 2 Gew.-%, insbesondere von 0,1 bis 1 Gew.-%, jeweils bezogen auf die anwendungsbereite Mischung, enthalten ist.

Eine weitere Verbesserung der pflegenden Eigenschaften des Produktes konnte durch den Einsatz eines Extraktes, der aus und/oder mithilfe von Algen und/oder Plankton gewonnen wird, erzielt werden. Insbesondere der Feuchtigkeitshaushalt der Fasern als auch deren Glanz konnte durch diese Extrakte erheblich gesteigert werden. Unter "Extrakten, die aus und/oder mithilfe von Algen und/oder Plankton gewonnen werden" sind erfindungsgemäß Wirkstoffmischungen zu verstehen, die entweder durch Extraktion von Algen und/oder Plankton selbst oder durch Extraktion der die Algen und/oder das Plankton umgebenen Wasserphase gewonnen werden. Erfindungsgemäß bevorzugte Algen und/oder Planktonarten sind ausgewählt aus den Gattungen Haptophyta, Schlundgeißler (Cryptista), Euglenozoa, Dinozoa, Chlorarachniophyta, Goldalgen (Chrysophyta), Kieselalgen (Bacillariophyta, auch Diatomeen genannt), Braunalgen (Phaeophyta), Dinogellaten, Rotalgen (Rhodophyta), Grünalgen (Chlorophyta), Picobiliphyta sowie der Blaualgen (beispielsweise Oscillatoria und Spirulina). Besonders bevorzugt sind im Rahmen der vorliegenden Erfindung Extrakte aus vornehmlich in Süßwasser vorkommenden Blaualgen.

Hinsichtlich der Art und Weise, wie die erfindungsgemäßen Extrakte aus den Algen- und/oder Planktonbestandteilen gewonnen wird, bestehen prinzipiell keine Einschränkungen. Als Extraktionsmittel zur Herstellung der genannten Algenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt.

Blaualgenextrakte, die mittels einer Wasser/Propylenglykol-Mischung gewonnen werden, haben sich als besonders geeignet erwiesen. Es hat sich dabei als besonders geeignet erwiesen, wenn diese Extraktionsmittel in einem Verhältnis von 1:10 bis 10:1 eingesetzt werden.

Weiterhin kann es erfindungsgemäß bevorzugt sein, Extrakte einzusetzen, die vor der Anwendung zumindest teilweise entfärbt wurden. Dies kann beispielsweise durch Nutzung von Aktivkohle erfolgen. Ebenso ist möglich, in den erfindungsgemäßen Mitteln als Algen- oder Plankton-Extrakt die wässrige Aufzucht- beziehungsweise Kulturlösung von Algen oder Plankton einzusetzen. Dazu werden die Algen oder das Plankton zunächst mit einer physikalischen Trennmethode, wie Filtration oder Zentrifugation von der Kulturlösung abgetrennt.

In einer besonders bevorzugten Ausführungsform der vorliegenden Erfindung enthält das Mittel einen Extrakt einer Blaualge, vorzugsweise einer Süßwasserblaualge, ganz besonders bevorzugt einer Blaualge der Gattung Spirulina.

Die erfindungsgemäßen Mittel enthalten die Algen- und/oder Planktonextrakte vorzugsweise in einer Menge von 0,001 bis 5 Gew.-%, vorzugsweise von 0,01 bis 2 Gew.-% bezogen auf das anwendungsbereite Mittel.

Weiterhin kann es erfindungsgemäß bevorzugt sein, wenn die Mittel zusätzlich mindestens eine Aminosäure und/oder mindestens ein Protein. Erfindungsgemäß bevorzugte Aminosäuren sind Arginin, Serin, Lysin, Glycin, Tyrosin, Prolin, Glutamin, Cystein und Histidin. Der Aminosäuren und/oder die Proteine ermöglicht eine überraschend stark ausfallende Strukturgebung der Haare.

Ferner konnten die Erfinder der vorliegenden Anmeldung zeigen, dass es sich vorteilhaft auf die Beschaffenheit des Schaums auswirkt, wenn die Mittel frei von Silikonölen formuliert sind.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide , insbesondere Arginin und/oder Serin; Proteinhydrolysate auf tierischer und/oder pflanzlicher Basis, wie beispielsweise Elastin-, Kollagen-, Keratin-, Seiden- und Milcheiweiß-Proteinhydrolysate, oder Mandel-, Reis-, Erbsen-, Kartoffel- und Weizenproteinhydrolysate, sowie in Form ihrer Fettsäure-Kondensationsprodukte oder gegebenenfalls anionisch oder kationisch modifizierten Derivate; pflanzliche Öle, wie Macadamianussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl und Teebaumöl; Lichtschutzmittel, wie derivatisierte Benzophenone, Zimtsäure-Derivate und Triazine; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen, insbesondere der Gruppen A, B₃, B₅, B₆, C, E, F und H; Pflanzenextrakte wie beispielsweise die Extrakte aus Aloe Vera, Angelica, Anis, Aprikose, Benzoe, Bergamotte, Birke, Brennnessel, Calmus, Cassis, Costus, Eibisch, Eichenrinde, Elemi, Estragon, Fichtennadel, Galbanum, Geranium, Ginseng, Grapefruit, Guajakholz , grünem Tee, Hamamelis, Hauhechel, Hopfen, Huflattich, Ingwerwurzel, Iris, Jasmin, Kamille, Kardamon, Klee, Klettenwurzel, Kiefer, Kiwi, Kokosnuss, Koriander, Kümmel, Latschen, Lavendel, Lemongras, Lilie, Limone, Lindenblüten, Litchi, Macis, Malve, Mandel, Mango, Melisse, Melone, Meristem, Myrrhe, Neroli, Olibanum, Opoponax, Orange, Patchouli, Petitgrain, Pinie, Quendel, Rooibos, Rosen, Rosmarin, Rosskastanie, Sandelholz, Salbei, Schachtelhalm, Schafgarbe, Sellerie, Tanne, Thymian, Wacholder, Weinblättern, Weißdorn, Weizen, Wiesenschaumkraut, Ylang-Ylang, Zeder und Zitrone; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -di-stearat sowie PEG-3-distearat; Pigmente sowie Treibmittel wie Propan-Butan-Gemische, N₂O Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht der Anwendungsmischung, eingesetzt.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Die Gegenwart von Oxidationsfarbstoffvorprodukten ist demnach keine zwingende Voraussetzung für einen Einsatz von Oxidationsmitteln in den erfindungsgemäßen Mitteln. Als Oxidationsmittel kommen Persulfate, Chlorite und insbesondere Wasserstoffperoxid oder dessen Anlagerungsprodukte an Harnstoff, Melamin sowie Natriumborat in Frage.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind Metallionen, Iodide, Chinone oder bestimmte Enzyme.

Bei einer Anwendung von Oxidationsmitteln wird das eigentliche Färbemittel zweckmäßigerweise unmittelbar vor der Anwendung durch Mischung der Zubereitung des Oxidationsmittels mit der Zubereitung, enthaltend die Verbindungen das mindestens eine Oxidationsfarbstoffvorprodukt, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Bei den pH-Werten im Sinne der vorliegenden Erfindung handelt es sich um pH-Werte, die bei einer Temperatur von 22 °C gemessen wurden.

Erfindungsgemäß bevorzugt ist auch der Einsatz von so genannten Komplexbildnern. Komplexilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt. In diesem Falle werden also normalerweise gestreckte Verbindungen durch Komplexbildung über ein Ion zu Ringen geschlossen. Die Zahl der gebundenen Liganden hängt von der Koordinationszahl des zentralen Ions ab. Gebräuchliche und im Rahmen der vorliegenden Erfindung bevorzugte Chelatkomplexbilder sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Erfindungsgemäß bevorzugte Komplexbildner sind Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz. Auch Dipicolinsäure wird erfindungsgemäß vorzugsweise als Komplexbildner eingesetzt. Mittel, die eine Kombination aus einem EDTA-Salz und HEDP und Dipicolinsäure enthalten sind erfindungsgemäß besonders bevorzugt.

Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Neben den bereits beschriebenen und zwingend enthaltenen Alkanolaminen kann das Mittel weitere Alkalisierungsmittel enthalten. Erfindungsgemäße, anorganische Alkalisierungsmittel werden bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat. Die als erfindungsgemäßes Alkalisierungsmittel einsetzbaren basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin, D/L-Arginin als ein Alkalisierungsmittel im Sinne der Erfindung eingesetzt. Schließlich ist ein weiteres bevorzugtes Alkalisierungsmittel Ammoniak. Bevorzugt werden die weiteren Alkalisierungsmittel in einer Menge von 0,05 bis 10 Gew.-%, insbesondere von 0,5 bis 5 Gew.-%, jeweils bezogen auf das Gesamtgewicht des anwendungsbereiten Mittels, enthalten.

In einer besonders bevorzugten Ausführungsform werden die erfindungsgemäßen Mittel dabei frei von Ammoniak als Alkalisierungsmittel formuliert. "Frei von Ammoniak" bedeutet dabei erfindungsgemäß, dass der Ammoniakgehalt der erfindungsgemäßen Mittel unterhalb von 1 Gew.-%, vorzugsweise unterhalb von 0,5 Gew.-%, insbesondere unterhalb von 0,1 Gew.-%, jeweils bezogen auf das anwendungsbereite Mittel, liegt.

Weiterhin hat es sich als besonders bevorzugt erwiesen, wenn die resultierende Applikationsflüssigkeit dünnflüssig formuliert ist. Applikationsflüssigkeiten, die nach Mischung mit der Oxidationsmittelzubereitung eine Viskosität von 0 bis 2000 mPas aufweisen (gemessen bei 22°C im Brookfield-Viskosimeter Typ RV-T mit Spindel LV-1 beziehungsweise RV-1 und einer Geschwindigkeit von 30U/min), haben sich als besonders bevorzugt erwiesen. Eine Viskosität von 0 bis 1000mPas, gemessen unter den genannten Bedingungen, ist erfindungsgemäß besonders bevorzugt. Eine Viskosität von 5 bis 500 mPas, insbesondere von 10 bis 50 mPas (gemessen untern den oben genannten Bedingungen) ist erfindungsgemäß ganz besonders bevorzugt.

Es ist erfindungsgemäß bevorzugt, wenn die beschriebene Haarfärbezubereitung in einem geeigneten Spender aufgenommen und zur jeweiligen Verwendung abgegeben wird. Dabei erfolgt die Ausgabe der Haarfärbezubereitung grundsätzlich in Schaumform. Die schaumförmige Konsistenz der Zubereitung ist in diesem Zusammenhang sehr breit zu verstehen und umfasst jedwede Mischung von einer fließfähigen Zubereitung und einer gasförmigen Komponente. Insofern sind sowohl fließfähige als auch im wesentlichen feste, stabile Schaumkonsistenzen vom Erfindungsgegenstand umfasst.

Grundsätzlich umfasst ein erfindungsgemäßer Spender zumindest einen Vorratsbehälter zur Aufnahme wenigstens einer Komponente der Haarfärbezubereitung und eine Applikationsvorrichtung zur Ausgabe der Haarfärbezubereitung in Form von Schaum. Dabei ist der Vorratsbehälter insbesondere als tuben- oder flaschenförmiger Behälter gestaltet, während die Applikationsvorrichtung diesen einseitig offenen Behälter verschließt. Die eigentliche Zubereitungsabgabe wird bevorzugt mittels einer geeigneten Druckquelle bewirkt, welche in den Spender, insbesondere den Vorratsbehälter, integriert ist, oder aber mittels manuellem Druckaufbau durch den Benutzer der Haarfärbezubereitung selbst veranlasst.

Als Beispiel für erfindungsgemäße Spender mit integrierter Druckquelle sind Druckbehälter zu nennen, die üblicherweise im Behälterinneren entweder einen geeigneten Druckspeicher aufweisen, z. B. einen mechanischen, oder aber ein Treibmittel enthalten und auf diesem Wege das Innere des Behälters unter Druck setzen. Derartige Druckbehälter verfügen üblicherweise über geeignete Ventilvorrichtungen zur Ausgabe der im Inneren des Druckbehälters befindlichen Zubereitung während einer entsprechenden Ventilbetätigung. Solche Druckbehälter sind vor allem in Verbindung mit gasförmigen und/oder flüssigen Treibmitteln in Form von Aerosolspendern für unterschiedlichste kosmetische Anwendungen vorbekannt, z. B. Haarstylingspray, Haarfärbezubereitungen, Deospray, Rasierschaum/-gel etc..

Alternativ können erfindungsgemäß auch manuell betätigte Spender zum Einsatz kommen, die sich lediglich der Krafteinwirkung des Nutzers bedienen, um eine schaumförmige Zubereitungsabgabe zu bewirken. Bei diesen Bauformen kann vorteilhaft auf eine zusätzliche Druckquelle, z. B. Treibmittel, verzichtet werden, was vor allem aus Kosten- sowie Nachhaltigkeitsgründen wünschenswert ist. Solche durch manuelle Krafteinwirkung betätigbaren Schaumspender sorgen neben der Förderung der Haarfärbezubereitung aus dem Vorratsbehälter zur Abgabeöffnung hin parallel auch für eine entsprechende Aufschäumung der Haarfärbezubereitung. Während dieser Aufschäumung oder Schaumbildung wird grundsätzlich die Haarfärbezubereitung mit einer gasförmigen Komponente, insbesondere Luft, vermischt. Im Einzelnen ist dazu eine Schäumvorrichtung vorgesehen, die dies bewerkstelligt.

Gemäß einer ersten Variante eines manuell betätigbaren Spenders ist dieser als Schüttelspender ausgeführt, mit mindestens einem Vorratsbehälter zur Aufnahme der Haarfärbezubereitung und einer zugehörigen Abgabevorrichtung zur schaumförmigen Abgabe der Haarfärbezubereitung. Dabei ist die Abgabevorrichtung, insbesondere lösbar, mit dem Vorratsbehälter verbunden. Die eigentliche Schaumbildung erfolgt innerhalb des Schüttelspenders durch Agitation der Haarfärbezubereitung innerhalb des Vorratsbehälters. Insofern bildet der Schüttelspender in Verbindung der entsprechenden Spenderbewegung die oben genannte Schäumvorrichtung. Im Anschluss an diese Art der Aufschäumung kann dann die Abgabe der schaumförmigen Haarfärbezubereitung mittels der Abgabevorrichtung erfolgen.

Eine weitere sinnvolle Spendervariante ergibt sich durch Ausgestaltung als Quetsch- oder Squeeze-Schaumspender. Ein solcher Quetsch-Schaumspender besitzt neben dem mindestens einen Vorratsbehälter zur Aufnahme der Haarfärbezubereitung eine entsprechende Applikationsvorrichtung, innerhalb der die Aufschäumung sowie anschließende Abgabe der Haarfärbezubereitung erfolgt. Die eigentliche Förderung der Haarfärbezubereitung aus dem Vorratsbehälter wird mittels Krafteinwirkung auf die flexible Vorratsbehälterwandung bewirkt. Dabei sorgt die reversible Verformung der Vorratsbehälterwandung für einen Druckaufbau im Inneren des Vorratsbehälters, so dass als Folge die Haarfärbezubereitung aus dem Vorratsbehälter getrieben wird. Dazu ist es notwendig die Vorratsbehälterwandung hinreichend flexibel bzw. reversibel verformbar zu gestalten. Dies wird gewährleistet durch eine anwendungsbezogen zielgerichte Auslegung der Dicke der Vorratsbehälterwandung in Verbindung mit einer geeigneten Materialauswahl für die Vorratsbehälterwandung. Bevorzugt wird die Vorratsbehälterwandung eines entsprechenden Quetsch-Schaumspenders aus einem Polyolefin, wie beispielsweise Polypropylen (PP), high density Polyethylen (HDPE), medium density Polyethylen (MDPE), low density Polyethylen (LDPE), linear low density Polyethylen (LLDPE), ausgeführt. Darunter ist Polypropylen (PP) bevorzugt geeignet.

Die Applikationsvorrichtung eines solchen Quetsch-Schaumspenders umfasst zur Aufschäumung der Haarfärbezubereitung auch eine entsprechende Schäumvorrichtung. Die Schäumvorrichtung ist in der Lage eine Zubereitungsmenge mit einer Gasmenge im geeigneten Dosierverhältnis zu vermischen, um die gewünschte Schaumkonsistenz der Haarfärbezubereitung auszubilden. Üblicherweise wird hierzu ein eingezogener Zubereitungsstrom mit einem eingezogenen Gasstrom innerhalb einer Mischkammer der Schäumvorrichtung zusammen geführt und dort durch strömungstechnische Verwirbelung vermengt. Besonders bevorzugt wird als gasförmige Komponente zur Schaumbildung Luft verwendet, die entweder unmittelbar aus der Vorratsbehälter oder der Umgebung eingezogen wird.

Die grundsätzliche Funktionsweise derartiger Quetsch-Schaumspender wird auch in den Dokumenten WO 2007/086730 A2/A3 sowie EP 1237660 B1 beschrieben. Ein erfindungsgemäßer Quetsch-Schaumspender kann auch entsprechend zu diesen Patentdokumenten ausgebildet sein. Insbesondere kann der erfindungsgemäße Quetsch-Schaumspender gemäß der. Offenbarung von EP 1237660 B1 so ausgeführt werden, dass eine Verwendung des Quetsch-Schaumspenders in im wesentlichen aufrechter Position als auch in Überkopfstellung möglich ist.

Analog kann der Spender auch als Pump-Schaumspender ausgebildet sein mit mindestens einer Vorratsbehälter zur Aufnahme der Haarfärbezubereitung sowie einer Applikationsvorrichtung, wobei in diesem Fall die Applikationsvorrichtung eine Pumpvorrichtung zur Förderung sowohl der Haarfärbezubereitung als der gasförmigen Komponente, bevorzugt Luft, aufweist und darüber hinaus eine entsprechende Schäumvorrichtung umfasst. Im Detail ergibt sich die Funktionsweise und der konstruktive Aufbau derartiger Pump-Schaumspender u. a. auch aus den Patentdokumenten WO 2007/083206 A1 oder WO 2007/091882 A1. Insbesondere kann der erfindungsgemäße Pump-Schaumspender gemäß der Offenbarung dieser genannten Dokumente ausgebildet sein.

Bei Verwendung der genannten Spendervarianten in Verbindung mit mehrkomponentigen Haarfärbezubereitungen, mit untereinander unverträglichen Einzelkomponenten (z. B. mehrkomponentige oxidative Haarfärbezubereitungen), muss sichergestellt werden, dass die Einzelkomponenten bis zur eigentlichen Zubereitungsanwendung getrennt bevorratet werden. Dies geschieht vorteilhaft durch Verwendung mehrerer Vorratsbehälter zur Aufnahme der jeweiligen Einzelkomponenten der Haarfärbezubereitung, wobei jeder Vorratsbehälter zur Abgabe der Haarfärbezubereitung jeweils in Fluidverbindung mit der zugehörigen Applikationsvorrichtung steht. Damit erfolgt eine Vermischung der Einzelkomponenten der Haarfärbezubereitung erst unmittelbar bei der Ausgabe der Zubereitung aus dem erfindungsgemäßen Spender bzw. bei der eigentlichen Zubereitungsverwendung. Beispielsweise kann ein erfindungsgemäßer Pump-Schaumspender auch mit zwei oder mehreren Vorratsbehältern für mehrere Zubereitungskomponenten ausgestattet sein. Der konstruktive Aufbau sowie die Funktionsweise eines derartigen Pump-Schaumspenders mit mehreren Vorratsbehältern wird u. a. auch in den Patentdokumenten WO 03/078075 A1 und WO 2005/102539 A1 im Detail beschrieben. Ein erfindungsgemäßer Pump-Schaumspender kann auch entsprechend der Offenbarung dieser Dokumente ausgeführt sein. Insbesondere ist es denkbar, den erfindungsgemäßen Pump-Schaumspender mit mehreren Vorratsbehältern und entsprechend mit mehreren Pumpvorrichtungen auszustatten, so dass für jede einzelne Zubereitungskomponente ein eigener Vorratsbehälter sowie eine eigene Pumpvorrichtung zur Verfügung stehen. In einem solchen Fall werden die Einzelkomponenten zur Zubereitungsabgabe mittels der mehreren Pumpen separat gefördert. Eine Mischung der Einzelkomponenten kann dann vor oder während der Aufschäumung der gesamten Haarfärbezubereitung erfolgen. Dies hat den Vorteil, dass lediglich eine Schäumvorrichtung für alle Einzelkomponenten erforderlich ist und der für die Aufschäumbarkeit der gesamten Haarfärbezubereitung notwendige Tensidgehalt nur einer der Einzelkomponenten beigegeben werden muss. Alternativ ist es aber auch denkbar mehrere Schäumvorrichtungen vorzusehen und damit jede geförderte Einzelkomponenten der Haarfärbezubereitung separat aufzuschäumen um erst im Anschluss daran eine Durchmischung der aufgeschäumten Einzelkomponenten zu bewirken. Dies erfordert in der Konsequenz separat aufschäumbare Einzelkomponenten, so dass in jeder Einzelkomponenten der für die Aufschäumbarkeit notwendige Tensidanteil enthalten sein muss.

Andererseits kann eine mehrkomponentige Haarfärbezubereitung erfindungsgemäß auch mit einer der oben beschriebenen Spendervarianten mit nur einem Vorratsbehälter und einer Applikationsvorrichtung angewendet werden. Dafür ist der Vorratsbehälter derart gestaltet, dass er sich wieder verschließbar öffnen lässt. Idealerweise ist der Vorratsbehälter mittels der Applikationsvorrichtung verschlossen, wobei die Applikationsvorrichtung lösbar mit dem Vorratsbehälter verbunden ist, beispielsweise über eine Schraub- oder Rastverbindung. Dies eröffnet die Möglichkeit den Vorratsbehälter bereits bei der Herstellung mit einer Komponente der Haarfärbezubereitung vorzukonfektionieren und weitere Komponenten der Haarfärbezubereitung erst kurz vor der eigentlichen Anwendung der Haarfärbezubereitung in den Vorratsbehälter zuzugeben. Hierbei sind die weiteren Komponenten der Haarfärbezubereitung dem gesamten Haarfärbeprodukt in Form eines Kit innerhalb von geeigneten separaten Behältern beigegeben und werden vom Nutzer unmittelbar vor der Anwendung der Haarfärbezubereitung im Vorratsbehälter gemischt.

Für alle aufgeführten Spendervarianten kann innerhalb der jeweiligen Applikationsvorrichtung die Durchmischung der Einzelkomponenten der Haarfärbezubereitung erleichtert bzw. verbessert werden, durch Verwendung geeigneter zusätzlicher Mischvorrichtungen, z. B. statischer Mischer (wie auch in WO 2005/102539 A1 beschrieben) oder poröser Einsatzelemente. Derartige Mischvorrichtungen lassen sich vorteilhaft an geeigneter Stelle innerhalb eines Strömungskanals für die Haarfärbezubereitung in der Applikationsvorrichtung anordnen.

Darüber hinaus können zusätzlich ein oder mehrere poröse Einsatzelemente eingesetzt werden, um die innerhalb der Schäumvorrichtung erzielbare Schaumkonsistenz positiv zu beeinflussen. Solche porösen Einsatzelemente sind beispielsweise schwamm- oder netzartig gestaltet und sind innerhalb der Schäumvorrichtung an geeigneter Stelle im Strömungskanal für die Haarfärbezubereitung positioniert, etwa unmittelbar stromaufwärts der Spenderabgabeöffnung, Das poröse Einsatzelement gestattet somit die Durchströmung der Haarfärbezubereitung und sorgt infolge strömungstechnischer Verwirbelung für eine feinere und homogenere Schaumkonsistenz. In Abhängigkeit von der jeweiligen Gestaltung des porösen Einsatzelementes lässt sich somit die Schaumkonsistenz direkt beeinflussen. Bei Verwendung eines netzartigen Einsatzelementes erweist es sich als sinnvoll das netzartige Einsatzelement bevorzugt mit einer Lochdichte von 50 bis 220 mesh (mesh = Anzahl der Löcher pro Inch), besonders bevorzugt von 90 bis 200 mesh, ganz besonders bevorzugt von 125 bis 175 mesh auszubilden. Bei Verwendung mehrerer netzartiger Einsatzelemente können auch Einsatzelemente mit unterschiedlichen Lochdichten zum Einsatz gelangen. Hierbei weist das erste, stromaufwärts positionierte netzartige Einsatzelement bevorzugt eine Lochdichte von 50 bis 220 mesh (mesh = Anzahl der Löcher pro Inch), besonders bevorzugt von 90 bis 200 mesh, ganz besonders bevorzugt von 125 bis 175 mesh auf. Das zweite, stromabwärts positionierte Netz weist bevorzugt eine Lochdichte von 160 bis 280 mesh, besonders bevorzugt von 175 bis 245 mesh und ganz besonders bevorzugt von 180 bis 220 mesh, auf. Letztlich wird die Anzahl der verwendeten porösen Einsatzelemente wie auch deren spezifische Lochdichte bzw. deren Porösitätseigenschaften in Abhängigkeit vom jeweiligen Anwendungsfall zielgerichtet ausgelegt.

Die Anwendungstemperaturen des resultierenden Schaums können in einem Bereich zwischen 15 und 40 °C liegen. Nach einer Einwirkungszeit von 5 bis 45 Minuten wird das Haarfärbemittel durch Ausspülen von dem zu färbenden Haar entfernt.

Ein zweiter Gegenstand der vorliegenden Erfindung ist ein Verfahren zur Färbung keratinischer Fasern, bei dem ein erfindungsgemäßes Mittel aus einem Spender als Schaum ausgebracht wird, dieser auf den Fasern verteilt wird, dann für eine Zeit von 1 bis 60 min, vorzugsweise von 5 bis 40 min, auf den Fasern verbleibt und anschließend aus den Fasern ausgewaschen wird.

Dabei kann der ausgebrachte Schaum unmittelbar auf den Haaransatz aufgetragen und anschließend mit den Händen oder einem mechanischen Hilfsmittel auf den Fasern verteilt werden. Es ist aber auch denkbar, dass der Schaum zunächst auf ein mechanisches Hilfsmittel, wie einen Kamm, aufgebracht wird und anschließend mit dessen Hilfe auf den Fasern verteilt wird. Unabhängig von der Art der Auftragung kann es erfindungsgemäß bevorzugt sein, wenn der Schaum anschließend in die Haare einmassiert wird. Es ist erfindungsgemäß bevorzugt, wenn die Applikationszubereitung bis unmittelbar vor der Anwendung als 2-Komponentensystem konfektioniert ist und erst bei der Anwendung durch Mischung der Komponenten hergestellt wird.

Dementsprechend ist es bevorzugt, wenn das erfindungsgemäße Mittel, enthaltend mindestens ein Oxidationsfarbstoffvorprodukt, mindestens ein Alkanolamin sowie mindestens ein zwitterionisches Tensid der Formel (I), unmittelbar vor der Anwendung durch Mischung eines ersten Mittels (A), enthaltend das mindestens eine Oxidationsfarbstoffvorprodukt, und eines zweiten Mittels (B), enthaltend mindestens ein Oxidationsmittel, erhalten wird.

Bezüglich weiterer bevorzugter Ausführungsformen der erfindungsgemäßen Verfahren gilt mutatis mutandis das zu den erfindungsgemäßen Mitteln Gesagte.

Besonders bevorzugt ist es, die Ausfärbung durch physikalische Maßnahmen zu unterstützen. Erfindungsgemäße Verfahren, bei denen die Anwendung durch Einwirkung von Wärme, IR- und/oder UV-Strahlung während der Einwirkzeit unterstützt wird.

Ein weiterer Gegenstand der vorliegenden Erfindung ist ein Kit zur Färbung keratinischer Fasern, das zwei getrennt voneinander konfektionierte Mittel aufweist, wobei das erste Mittel (A) mindestens ein Oxidationsfarbstoffvorprodukt enthält, das zweite Mittel (B) mindestens ein Oxidationsmittel enthält, und die Mischung der Mittel (A) und (B) ein Mittel, enthaltend mindestens ein Oxidationsfarbstoffvorprodukt, mindestens ein Alkanolamin sowie mindestens ein zwitterionisches Tensid der Formel (I), ergibt. Besonders bevorzugt ist ein Kit, das weiterhin einen Spender enthält, der geeignet ist, die Mischung der Mittel (A) und (B) in Form eines Schaums abzugeben.

Bezüglich weiterer bevorzugter Ausführungsformen des erfindungsgemäßen Kits gilt mutatis mutandis das zu den erfindungsgemäßen Mitteln Gesagte.

### Beispiele

Es wurden die folgenden Zubereitungen hergestellt. Die Mengenangaben verstehen sich, soweit nichts anderes vermerkt ist, jeweils in Gewichtsprozent.

**1 Färbelösungen**

| Rohstoff | Erfindungsgemäße Färbelösung F1 | Nicht-erfindungsgemäße Färbelösung F2 |
|---|---|---|
| Plantacare^{®} 818 UP | 25,00 | 20,00 |
| Dehyton^{®} K | -- | 26,00 |
| Genagen^{®} KB | 30,00 | -- |
| Cremophor^{®} CO 60 | 3,00 | 3,00 |
| Tetranatrium-EDTA | 0,20 | 0,20 |
| p-Toluylendiaminsulfat | 2,31 | 2,31 |
| 2-Amino-4-(2-hydroxyethyl)aminoanisol-sulfat | 0,03 | 0,03 |
| Resorcin | 0,89 | 0,89 |
| m-Aminophenol | 0,26 | 0,26 |
| Natriumsulfit | 0,20 | 0,20 |
| Ascorbinsäure | 0,05 | 0,05 |
| Monoethanolamin | 10,00 | 10,00 |
| Eau Vitale d'algue bleue^{®} | 2,0 | 2,0 |
| Parfum | qs | qs |
| Wasser | ad 100 | ad 100 |

**2 Entwicklerzubereitungen**

| Rohstoff | E1 | E1-DK |
|---|---|---|
| Dipicolinsäure | 0,10 | 0,10 |
| Dinatriumpyrophosphat | 0,03 | 0,03 |
| Aculyn^{®} 33A | 2,50 | 2,50 |
| Texapon^{®} NSO | 2,00 | 2,00 |
| Cremophor^{®} CO 60 | -- | 2,00 |
| Eumulgin^{®} L | -- | 0,40 |
| Natriumhydroxid (wässrig, 45%ig) | 0,73 | -- |
| Turpinal^{®} SL | 1,50 | 1,50 |
| Wasserstoffperoxid (wässrig, 50 %ig) | 15,20 | 15,20 |
| Wasser | ad 100 | ad 100 |

### 3 Verzeichnis der eingesetzten Handelsprodukte

- Aculyn^{®} 33A: ca. 28% Festkörper in Wasser; INCI-Bezeichnung: Acrylates Copolymer
- Cremophor^{®} CO60: INCI-Bezeichnung: PEG-60 Hydrogenated Castor Oil (BASF)
- Dehyton^{®} K: ca. 30% Aktivsubstanz; INCI-Bezeichnung: Aqua (Water), Cocamidopropyl Betaine (Cognis)
- Eau Vitale d'algue bleue^{®}: ca. 0,1-0,99 Gew.-% Aktivsubstanzgehalt; INCI-Bezeichnung: Aqua (Water), Plankton Extract, Penoxyethanol (Soliance)
- Eumulgin^{®} L: INCI-Bezeichnung: PPG-1-PEG-9 Lauryl Glycol Ether (Cognis)
- Plantacare^{®} 818UP: ca. 51-53% Aktivsubstanzgehalt; INCI-Bezeichnung: Coco-Glucoside, Aqua (Water) (Cognis)
- Texapon^{®} NSO: ca. 27,5% Aktivsubstanz; INCI-Bezeichnung: Sodium Laureth Sulfate (Cognis)
- Turpinal^{®} SL: ca. 58 - 61% Aktivsubstanzgehalt; INCI-Bezeichnung: Etidronic Acid, Aqua (Water) (Solutia)

### 4 Vergleichsversuche

Die beiden Färbelösungen F1 und F2 wurden die dem Fachmann geläufige Methode hergestellt. Dabei zeigte sich, dass die Färbezubereitung F2 nach kurzer Zeit einen intensiven Amin-artigen Geruch erzeugte, der von der überwiegenden Zahl an Testpersonen als ausgesprochen unangenehm beschrieben wurde. Dieser Geruch blieb auch nach Vermischen mit einer der Entwicklerzubereitungen E1 oder E1-DK bestehen. Die erfindungsgemäße Färbezubereitung F1 hingegen wies für sich oder auch nach Vermischen mit den Entwicklerzubereitungen keinerlei unerwünschte Geruchsentwicklung auf.

### 5 Ausfärbungen

### 5.1 Anwendung mit einem Quetsch-Schaumspender

60ml der Oxidationsmittelzubereitung E1 wurde in einem Quetsch-Schaumspender vorgelegt. Unmittelbar vor der Anwendung wurden 60ml der erfindungsgemäßen Färbelösung F1 vorsichtig zugegeben, so dass keine Schaumbildung erfolgte. Anschließend wurde die Squeezeflasche mehrfach vorsichtig auf den Kopf und wieder zurückgedreht, so dass eine gründliche Durchmischung der Komponente bei geringer Schaumbildung stattfand. Die resultierende Anwendungszubereitung hatte eine Viskosität von 15 mPas (gemessen bei 22°C im Brookfield-Viskosimeter Typ RV-T mit Spindel LV-1 und einer Geschwindigkeit von 30U/min) und wies einen pH-Wert von 9,5 auf.

Der Schaum wurde durch Drücken des Quetsch-Schaumspenders direkt auf den Haaransatz ausgebracht, anschließend die Haarlängen benetzt und zum Schluss mit einem Kamm gleichmäßig in den Fasern verteilt. Nach einer Einwirkzeit von 30 min bei Raumtemperatur wurden die Haare gründlich mit Wasser gespült, shampooniert und mit einem Haartrockner getrocknet.

Die Haare waren satt schwarzbraun gefärbt, zeigten eine perfekte Farb-Egalisierung von Wurzel bis Haarspitze und einen hervorragenden Glanz.

### 5.2 Anwendung mit Pump-Schaumspender

60 ml der Oxidationsmittelzubereitung E1 wurde in einem Pump-Spender vorgelegt. Unmittelbar vor der Anwendung wurden 60 ml der Färbemittelzubereitung F1 vorsichtig zugegeben, so dass keine Schaumbildung erfolgte. Anschließend wurde der Pump-Spender mehrfach vorsichtig auf den Kopf und wieder zurückgedreht, so dass eine gründliche Durchmischung der Komponente bei geringer Schaumbildung stattfand. Die resultierende Anwendungszubereitung hatte eine Viskosität von 12 mPas (gemessen bei 22°C im Brookfield-Viskosimeter Typ RV-T mit Spindel LV-1 und einer Geschwindigkeit von 30 U/min) und wies einen pH-Wert von 9,5 auf.

Der Schaum wurde durch Benutzung des Pumpkopfes zunächst auf die Handinnenfläche und anschließend auf den Haaransatz und die Haarlängen ausgebracht, zum Schluss mit einem Kamm gleichmäßig in den Fasern verteilt. Nach einer Einwirkzeit von 30 min bei Raumtemperatur wurden die Haare gründlich mit Wasser gespült, shampooniert und mit einem Haartrockner getrocknet.

Die Haare waren satt schwarzbraun gefärbt, zeigten eine perfekte Farb-Egalisierung von Wurzel bis Haarspitze und einen hervorragenden Glanz.

### 5.3 Anwendung mit einem Pump-Schaumspender mit mehreren Vorratsbehältern

60 ml der Oxidationsmittelzubereitung E1-DK und 60 ml der Färbemittelzubereitung F1 wurden in den beiden Vorratskammern eines Pump-Schaumspenders mit zwei Vorratsbehältern vorgelegt. Die durch Pumpen entstehende resultierende Anwendungszubereitung wies einen pH-Wert von 9,5 auf.

Der Schaum wurde durch Benutzung der gekoppelten Pumpköpfe zunächst aus dem Pump-Schaumspender auf die Handinnenfläche und anschließend auf den Haaransatz und die Haarlängen ausgebracht, zum Schluss mit einem Kamm gleichmäßig in den Fasern verteilt. Nach einer Einwirkzeit von 30 min bei Raumtemperatur wurden die Haare gründlich mit Wasser gespült, shampooniert und mit einem Haartrockner getrocknet

Die Haare waren satt schwarzbraun gefärbt, zeigten eine perfekte Farb-Egalisierung von Wurzel bis Haarspitze und einen hervorragenden Glanz.

## Patentansprüche

1. Mittel zum Färben keratinischer Fasern, enthaltend in einem kosmetisch akzeptablen Träger mindestens ein Oxidationsfarbstoffvorprodukt sowie mindestens ein Alkanolamin, **dadurch gekennzeichnet, dass**
das Mittel mindestens ein zwitterionisches Tensid der Formel (I), 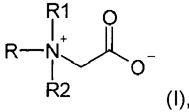 worin
R für eine gesättigte oder ungesättigte C₁₀-C₂₀-Alkylkette steht und
R1 und R2 jeweils unabhängig voneinander für eine C₁-C₄-Alkylgruppe oder eine C₂-C₄-Hydroxyalkylgruppe stehen, und
mindestens ein nichtionisches Tensid enthält, wobei
das Mittel als nichtionisches Tensid mindestens ein Alkylpolyglucosid und/oder Alkenylpolyglucosid enthält.

2. Mittel nach Anspruch 1, **dadurch gekennzeichnet, dass** es als Alkanolamin mindestens Monoethanolamin enthält.

3. Mittel nach einem der Ansprüche 1 oder 2, **dadurch gekennzeichnet, dass** es als zwitterionisches Tensid mindestens eine Verbindung der Formel (I) enthält, worin R1 und R2 jeweils für eine Methylgruppe und R für eine Cocosalkylgruppe stehen.

4. Mittel nach einem der Ansprüche 1 bis 3, **dadurch gekennzeichnet, dass** es ein oder mehrere zwitterionische Tenside der Formel (I) in einem Gesamtgewichtsanteil von mindestens 2,5 Gew.-%, bevorzugt von mindestens 3 Gew.-% und insbesondere bevorzugt von mindestens 4 Gew.-%, bezogen auf das Gesamtgewicht des anwendungsbereiten Mittels, enthält.

5. Mittel nach Anspruch 1, **dadurch gekennzeichnet, dass** das Mittel als nichtionisches Tensid mindestens ein ethoxyliertes Tensid mit mindestens 30 Ethylenoxideinheiten enthält.

6. Mittel nach einem der Ansprüche 1 oder 5, **dadurch gekennzeichnet, dass** das Mittel als nichtionisches Tensid eine Kombination aus mindestens einem Alkylpolyglucosid und mindestens einem ethoxylierten Tensid mit mindestens 30 Ethylenoxideinheiten enthält.

7. Mittel nach einem der Ansprüche 1 bis 6, **dadurch gekennzeichnet, dass** das Mittel einen Gesamttensidgehalt von mindestens 10 Gew.-%, bevorzugt mindestens 12 Gew.-%, bezogen auf das Gesamtgewicht des anwendungsbereiten Mittel, aufweist.

8. Mittel nach einem der Ansprüche 1 bis 7, **dadurch gekennzeichnet, dass** das Mittel frei von Ammoniak formuliert ist.

9. Verfahren zur Färbung keratinischer Fasern, **dadurch gekennzeichnet, dass** ein Mittel nach einem der Ansprüche 1 bis 8 aus einem Spender als Schaum ausgebracht wird, dieser auf den Fasern verteilt wird, dann für eine Zeit von 1 bis 60 Minuten, vorzugsweise von 5 bis 40 Minuten, auf den Fasern verbleibt und anschließend aus den Fasern ausgewaschen wird.

10. Verfahren nach Anspruch 9, **dadurch gekennzeichnet, dass** das anwendungsbereite Mittel nach einem der Ansprüche 1 bis 8 unmittelbar vor der Anwendung durch Mischung eines ersten Mittels (A), enthaltend mindestens ein Oxidationsfarbstoffvorprodukt, und eines zweiten Mittels (B), enthaltend mindestens ein Oxidationsmittel, erhalten wird.

11. Kit zur Färbung keratinischer Fasern, **dadurch gekennzeichnet, dass** es mindestens zwei getrennt voneinander konfektionierte Mittel aufweist, wobei das erste Mittel (A) mindestens ein Oxidationsfarbstoffvorprodukt enthält, das zweite Mittel (B) mindestens ein Oxidationsmittel enthält, und die Mischung der Mittel (A) und (B) ein Mittel nach einem der Ansprüche 1 bis 8 ergibt.

12. Kit nach Anspruch 11, **dadurch gekennzeichnet, dass** weiterhin ein Spender enthalten ist, der geeignet ist, die Mischung der Mittel (A) und (B) in Form eines Schaums abzugeben.

## Claims

1. An agent for dying keratin fibers, containing, in a cosmetically acceptable carrier, at least one oxidation dye precursor and at least one alkanolamine **characterized in that** the agent contains at least one zwitterionic surfactant of the formula (I), 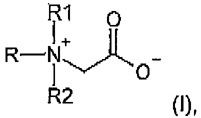 where
R represents a saturated or unsaturated C₁₀-C₂₀ alkyl chain, and
R1 and R2 each represent, independently of one another, a C₁-C₄ alkyl group or a C₂-C₄ hydroxyalkyl group, and
at least one non-ionic surfactant, the agent containing, as the non-ionic surfactant, at least one alkyl polyglucoside and/or alkenyl polyglucoside.

2. The agent according to claim 1, **characterized in that** said agent contains, as the alkanolamine, at least one monoethanolamine.

3. The agent according to one of claims 1 or 2, **characterized in that** said agent contains, as the zwitterionic surfactant, at least one compound of the formula (I), where R1 and R2 each represent a methyl group and R represents a coco alkyl group.

4. The agent according to one of claims 1 to 3, **characterized in that** said agent contains one or more zwitterionic surfactants of the formula (I) in an overall percentage by weight of at least 2.5 wt.%, preferably at least 3 wt.%, and particularly preferably at least 4 wt.%, based on the overall weight of the ready-to-use agent.

5. The agent according to claim 1, **characterized in that** the agent contains, as the non-ionic surfactant, at least one ethoxylated surfactant comprising at least 30 ethylene oxide units.

6. The agent according to one of claims 1 or 5, **characterized in that** the agent contains, as the non-ionic surfactant, a combination of at least one alkyl polyglucoside and at least one ethoxylated surfactant comprising at least 30 ethylene oxide units.

7. The agent according to one of claims 1 to 6, **characterized in that** the agent has an overall surfactant content of at least 10 wt.%, preferably at least 12 wt.%, based on the overall weight of the ready-to-use agent.

8. The agent according to one of claims 1 to 7, **characterized in that** the agent is formulated so as to be free from ammonia.

9. A method for dying keratin fibers, **characterized in that** an agent according to one of claims 1 to 8 is discharged from a dispenser as a foam, said foam is distributed on the fibers, then left on the fibers for 1 to 60 minutes, preferably 5 to 40 minutes, and then washed out of the fibers.

10. The method according to claim 9, **characterized in that** the ready-to-use agent according to one of claims 1 to 8 is obtained directly before use by mixing a first agent (A), containing at least one oxidation dye precursor, and a second agent (B), containing at least one oxidation agent.

11. A kit for dying keratin fibers, **characterized in that** said kit contains at least two separately packaged agents, the first agent (A) containing at least one oxidation dye precursor, the second agent (B) containing at least one oxidation agent and the mixture of agents (A) and (B) providing an agent according to one of claims 1 to 8.

12. The kit according to claim 11, **characterized in that** said kit also contains a dispenser suitable for dispensing the mixture of agents (A) and (B) in the form of a foam.

## Revendications

1. Agent de coloration de fibres kératiniques, comprenant dans un support cosmétiquement acceptable au moins un précurseur de colorant d'oxydation et au moins un alcool aminé, **caractérisé en ce que**
l'agent comprend au moins un tensioactif zwitterionique de formule (I), 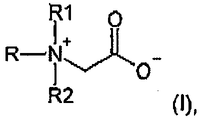 où
R représente une chaîne alkyle saturée ou insaturée en C₁₀ à C₂₀ et
R1 et R2 représentent indépendamment l'un de l'autre un groupe alkyle en C₁ à C₄ ou un groupe hydroxyalkyle en C₂ à C₄, et
au moins un tensioactif non ionique,
l'agent comprenant au moins un alkylpolyglucoside et/ou un alcénylpolyglucoside comme tensioactif non ionique

2. Agent selon la revendication 1, **caractérisé en ce qu'**il comprend au moins de la monoéthanolamine comme alcool aminé.

3. Agent selon l'une des revendications 1 ou 2, **caractérisé en ce qu'**il comprend comme tensioactif zwitterionique au moins un composé de formule (I), où R1 et R2 représentent chacun un groupe méthyle et R représente un groupe alkyle de coco.

4. Agent selon l'une quelconque des revendications 1 à 3, **caractérisé en ce qu'**il comprend un ou plusieurs tensioactifs zwitterioniques de formule (I) dans une proportion totale d'au moins 2,5 % en poids, de préférence d'au moins 3 % en poids et de manière davantage préférée d'au moins 4 % en poids, par rapport au poids total de l'agent prêt à l'emploi.

5. Agent selon la revendication 1, **caractérisé en ce que** l'agent comprend, comme tensioactif non ionique, au moins un tensioactif éthoxylé comportant au moins 30 motifs d'oxyde d'éthylène.

6. Agent selon l'une des revendications 1 ou 5, **caractérisé en ce que** l'agent comprend comme tensioactif non ionique une combinaison d'au moins un alkylpolyglucoside et au moins un tensioactif éthoxylé comportant au moins 30 motifs d'oxyde d'éthylène.

7. Agent selon l'une quelconque des revendications 1 à 6, **caractérisé en ce que** l'agent présente une teneur totale en tensioactifs d'au moins 10 % en poids, de préférence d'au moins 12 % en poids, par rapport au poids total de l'agent prêt à l'emploi.

8. Agent selon l'une quelconque des revendications 1 à 7, **caractérisé en ce que** l'agent est formulé sans ammoniaque.

9. Procédé de coloration de fibres kératiniques **caractérisé en ce qu'**un agent selon une des revendications 1 à 8 est pulvérisé sous forme de mousse à l'aide d'un distributeur, que cette mousse est répartie sur les fibres, puis qu'elle y reste pendant une durée allant de 1 à 60 minutes, de préférence de 5 à 40 minutes, et enfin qu'elle est enlevée des fibres par rinçage.

10. Procédé selon la revendication 9, **caractérisé en ce que** l'agent prêt à l'emploi selon une des revendications 1 à 8 est obtenu juste avant l'application par mélange d'un premier agent (A), comprenant au moins un précurseur de colorant d'oxydation, et d'un deuxième agent (B), comprenant au moins un agent d'oxydation.

11. Kit de coloration de fibres kératiniques **caractérisé en ce qu'**il présente au moins deux agents fabriqués séparément l'un de l'autre, le premier agent (A) comprenant au moins un précurseur de colorant d'oxydation, le deuxième agent (B) comprenant au moins un agent d'oxydation, et le mélange des agents (A) et (B) produisant un agent selon une des revendications 1 à 8.

12. Kit selon la revendication 11 **caractérisé en ce qu'**il comprend en outre un distributeur adapté pour distribuer le mélange des agents (A) et (B) sous forme de mousse.
